# EUROPEAN PATENT SPECIFICATION

(11) **EP 2 787 939 B1**
(45) Date of publication and mention of the grant of the patent: **27.03.2019**
(21) Application number: 12799147.9
(22) Date of filing: 06.12.2012
(51) Int. Cl.: A61F 5/445, A61B 5/107

(54) **OSTOMY MEASURING TOOL**
OSTOMIEMESSWERKZEUG
OUTIL DE MESURE DE STOMIE

(30) Priority: 08.12.2011 EP 11192522
(43) Date of publication of application: 15.10.2014
(73) Proprietor: Hollister Incorporated, Libertyville, IL 60048-3781 (US)
(72) Inventor: HANNESTAD, Vigdis, 5003 Bergen (NO); GRUT, Lara Meisen, 2900 Hellerup (DK); OLSEN, Kinn Toft, 3070 Snekkersten (DK)
(74) Representative: Høiberg P/S
(86) International application number: PCT/EP2012/074633
(87) International publication number: WO 2013/083689

(56) References cited:
- EP-A1- 0 790 048
- WO-A1-2006/075949
- WO-A1-2006/122565
- DE-U1- 20 012 498
- DE-U1- 20 307 688
- US-A1- 2004 111 072

## Description

The present disclosure relates to a measuring tool for assessing the stomal and/or peristomal configuration of a patient comprising a plurality of ostomy guide plates.

DE 200 12 498 U1 is regarded as the closest prior art and although it does not show a measuring tool it discloses a set of convex forms provided with a stepped outer surface as cutting guides for enlarging the stomal aperture.

### Background of the disclosure

Fundamental to the rehabilitation of persons with abdominal stomas is a dependable, secure pouching system. Achieving this will result in cost effectiveness and reduce complications such as leakage, odor, peristomal skin breakdown, pain and loss of self-esteem.

Various kinds of ostomy devices are known in the art. A two piece ostomy device typically includes a baseplate having two sides with an adhesive dressing on one side that adheres to the peristomal area of the patient's skin and a pouch which is removably affixed to the opposite side of the baseplate. Upon renewal only the pouch is removed from the baseplate and replaced. In a one piece ostomy device the baseplate and the pouch are welded together to form a single product. Upon renewal the whole appliance is removed from the skin and replaced.

However, ostomy pouch systems vary from manufacturer to manufacturer in style, shape, color and functionality and an addition there are a number of variations regarding skin barrier choices, tape or no tape, moldable styles and adhesively coupled configurations. It may therefore pose a challenge to healthcare professionals, such as nurses, to asses a patient's stomal and peristomal configuration in order to select the correct ostomy product to obtain a secure seal between the ostomy pouch and the patient. In order to estimate the basic dimensions of the stoma a planar display arrangement for ostomy care supplies as disclosed in US 2004/0111072 may be applied. This display arrangement includes a stoma measuring device, which includes circular holes of a number of different diameters to measure a stoma. Other devices for measuring various human anatomical features, wounds, or stomas are known in the art. Several of these devices are transparent or are constructed in a manner such that a user is able to look through the device and easily assess size. E.g. US 7,401,413 discloses a device for making dimensional measurements of a wound by means of two separate members for gauging 1) the area and 2) the depth of the wound. Further, US 6,764,453 discloses a stoma measuring device adapted for insertion into a stoma to measure the length of the stoma.

### Summary of the disclosure

Healthcare professionals may face situations where the stoma does not sufficiently protrude beyond the abdominal skin surface of the patient or where the muscles surrounding the stoma lack rigidity. In these situations a flat baseplate may not provide a fluid tight and weight supporting seal between stoma and pouch. The solution could be a convex baseplate secured around the stoma so as to apply pressure against the patient's abdomen thereby causing the stoma to protrude into the ostomy appliance. Ostomy pouching systems with convex baseplates have been commercially available for years for both one piece and two piece ostomy devices, but since these convex products were launched it has been a problem for the nurse to determine when to apply a convex ostomy solution and which configuration to select from the ostomy product range.

Accordingly, it is an object of the present disclosure to provide a solution for a healthcare professional to be able assess whether a flat or convex solution is the correct choice.

This object is achieved by the present disclosure which in a first aspect discloses an ostomy guide plate for assessing the stomal and/or peristomal configuration of a patient comprising a distal face, a proximal face with a convex protrusion and an aperture in said convex protrusion, wherein the ostomy guide plate is adapted for disposition on the skin of the patient with the aperture positioned over the stoma. In use, the nurse or practitioner positions the convex guide plate on the exterior of the patient's stoma with the proximal side of the convex guide plate facing the skin of the patient and by gently applying pressure to the guide plate the nurse can assess whether a convex solution is the proper choice. Thus, it is typically the proximal face of the ostomy guide plate that is adapted for direct contact with the abdominal skin of the patient.

A further object of the disclosure is to provide a solution for a healthcare professional to be capable of selecting the best possible convex solution for their patients.

This object is achieved by the present disclosure which in a second aspect relates to a measuring tool (aka a convex measuring tool) for assessing the stomal and/or peristomal configuration of a patient comprising a plurality of these ostomy guide plates wherein each plate is provided with at least one distinct dimensional parameter. In use, the nurse or practitioner positions the convex guide plates of the measuring tool in turn on the exterior of the patient's stoma and can thereby quickly and easily be able to assess the circumference and depth of the stoma thereby guiding him/her in selecting the proper size convex product for the patient's stoma.

The present disclosure further relates to the use of an ostomy guide plate for assessing the stomal and/or peristomal configuration of a patient, wherein the ostomy guide plate comprises a distal face, a proximal face with a convex protrusion and an aperture in said convex protrusion, and wherein the ostomy guide plate is adapted for disposition on the skin of the patient with the aperture positioned over the stoma.

As stated above the ostomy guide plate according to the present disclosure is adapted for disposition on the skin of the patient with the aperture positioned over the stoma. Preferably the present ostomy guide plate is adapted for non-binding disposition on the skin of the patient with the aperture positioned over the stoma. By gently pressing the guide plate such that the stoma possibly protrudes through the aperture, the medical professional can assess the necessary configuration of the ostomy solution for the patient. This typically requires that the guide plate is configured such that it can be comfortably placed on the skin of the patient and removed again without causing discomfort to the patient, i.e. the proximal side of the guide plate can be provided with a smooth non-binding surface, e.g. without adhesives or sharp edges or protrusions. Further, the surface profile of the convex protrusion may be a smooth or rounded curve to provide comfort to the patient. Convex pressure rings, e.g. disclosed in EP 0790048, used for bonding a baseplate and an ostomy pouch together also comprise a distal face, a proximal face with a convex protrusion and an aperture in said convex protrusion. However, these pressure rings are not adapted for disposition directly on the skin of the patient and can therefore not be used for assessing the stomal configuration of a patient.

The assessment of the stomal configuration may be improved if the convex guide plate is provided with see-through walls of the tool for the nurse to look through the plate during assessment. This tool will also guide the nurse to decide if paste/seals are needed in conjunction with the standard firm convex product chosen, to obtain the best possible result.

In a preferred embodiment, the plate, or at least a part of it, is transparent for a nurse to be able to see through the plate and asses the stomal and peristomal configuration. Alternatively the plate can be translucent. The distal side of the plate is preferably provided with a concave depression corresponding to the convex protrusion on the proximal face, thereby providing a substantially constant thickness of the plate. Furthermore, the convex guide plate thereby becomes stackable.

In the preferred embodiment the aperture is circular or elliptical. Furthermore, the convex protrusion is preferably circular or elliptical. The plate may also be substantially circular or elliptical.

In the preferred embodiment the convex protrusion and the aperture are concentrically arranged in the plate. The convex protrusion is preferably located substantially centrally in the plate. The aperture is preferably located substantially centrally in the convex protrusion. In the preferred embodiment the maximum diameter of the aperture is predefined. Further, the outer diameter of the plate is preferably predefined. The depth and/or the outer diameter of the convex protrusion may also be predefined. The ostomy guide plate may further comprise a colour coding corresponding to the predefined value of the aperture, the outer diameter, the depth of the convex protrusion, and/or the diameter of the convex protrusion, e.g. in the form of a visible coloured mark on the distal side of the guide plate. This colour code may then correspond to the colour code of one or more ostomy products with a configuration that correspond to the dimensions of the guide plate.

The convex protrusion is preferably provided with a substantially plane resting plateau. I.e. when the convex guide plate is positioned on the skin of the user, the convex guide plate will rest on the resting plateau and if pressure is applied to the distal face of the guide plate the pressure is directed to the skin via the resting plateau. The diameter of the resting plateau is preferably predefined. Such a plurality of geometric considerations is provided to mimic the physical configurations of the actual convex pouching systems.

In the preferred embodiment the ostomy guide plate comprises at least one visible mark for indicating at least one dimensional parameter of said ostomy guide plate. I.e. the diameter of the aperture may be indicated on the distal face of the plate visible to a nurse when the plate is in use, in order for the nurse to be able to assess the diameter of the stoma by comparing to the known diameter of the aperture.

In the preferred embodiment the plate is substantially rigid, in order to mimic the physical properties of the firm convex pouching systems. The ostomy guide plate can e.g. be manufactured in thermoplastics, such as ABS or polystyrene, or another nontoxic, washable material.

The ostomy guide plate may be provided with a plane distal face and a convex proximal face. Thus, the guide plate can be handled by using the plane surface as a handle. However, the ostomy guide plate may also be provided with one or more dedicated handles, e.g. extending from its perimeter. E.g. two handles located oppositely at the plate that makes it possible to distribute applied pressure equally to the plate.

As previously stated convex products may be applied if the stoma does not sufficiently protrude beyond the abdominal skin surface of the patient or where the muscles surrounding the stoma lack rigidity. The function of a convex baseplate is then to apply pressure against the patient's abdomen thereby causing the stoma to protrude into the ostomy appliance. The present ostomy guide plate can be provided to test this functionality, because the ostomy guide plate is preferably adapted such that pressure can be applied to the distal face of the plate, whereby the convex protrusion is pressed into the abdominal skin of the patient. The pressure can be applied via one or more handles attached to the plate or the pressure can be applied by directly pressing the distal face of the plate. The known convex baseplates are not suitable for assessing the stomal configuration of a patient because they are not adapted for disposition on the skin of the patient. Baseplates are provided with an adhesive layer such that they can be attached to the abdominal skin of the patient. The adhesive layer is initially covered and this cover is not adapted for disposition on the patient's skin. When the adhesive layer is exposed the baseplate is not adapted for non-binding disposition on the skin of the patient.

The ostomy guide plate may be adapted such that the entire proximal face of the plate can touch the skin of the patient when pressure is applied to the distal face of the plate.

The thickness of the guide plate is a balance between rigidity, transparency, weight and manageability. Thus, in one embodiment the thickness of the ostomy guide plate is between 0.1 and 5 mm, such as between 0.1 and 1 mm, such as between 1.2 and 1.4 mm, such as between 1.4 and 1.6 mm, such as between 1.6 and 1.8 mm, such as between 1.8 and 2.0 mm, such as between 2.0 and 2.2 mm, such as between 2.2 and 2.4 mm, such as between 2.4 and 2.6 mm, such as between 2.6 and 2.8 mm, such as between 2.8 and 3.0 mm, such as between 3 and 4 mm, such as between 4 and 5 mm.

The diameter of the aperture may be between 10 and 80 mm, such as between 10 and 15 mm, such as between 15 and 20 mm, such as between 20 and 25 mm, such as between 25 and 30 mm, such as between 30 and 35 mm, such as between 35 and 40 mm, such as between 40 and 45 mm, such as between 45 and 50 mm, such as between 50 and 55 mm, such as between 55 and 60 mm, such as between 60 and 65 mm, such as between 65 and 70 mm, such as 22 mm, 23 mm, 30 mm, 32 mm, 35 mm, 40 mm, 42 mm, 45 mm or 50 mm.

The depth of the convex protrusion is preferably between 1 and 10 mm, such as approx. 1, 2, 3, 4, 5, 6, 7, 8, 9 or 10 mm, most preferably 6 mm.

The outer diameter of the plate is preferably between 50 and 150 mm, such as between 60 and 70 mm, such as between 70 and 75 mm, such as between 75 and 80 mm, such as between 80 and 85 mm, such as between 85 and 90 mm, such as between 90 and 95 mm, such as between 95 and 100 mm, such as between 100 and 105 mm, such as between 105 and 110 mm, such as between 110 and 115 mm, such as between 115 and 120 mm, such as between 120 and 125 mm, such as between 125 and 130 mm, such as 78 mm, 80 mm, 86 mm, 88 mm, 93 mm, 98 mm, 100 mm, 106 mm, 113 mm, 116 mm or 121 mm.

The diameter of the resting plateau is preferably between 20 and 80 mm, such as between 20 and 25 mm, such as between 25 and 30 mm, such as between 30 and 35 mm, such as between 35 and 40 mm, such as between 40 and 45 mm, such as between 45 and 50 mm, such as between 50 and 55 mm, such as between 55 and 60 mm, such as between 60 and 65 mm, such as between 65 and 70 mm, such as between 70 and 75 mm, such as between 75 and 80 mm, such as 30 mm, 30.5 mm, 31 mm, mm, 37 mm, 37.5 mm, 38 mm, 42 mm, 49 mm, 49.5 mm, 50 mm, 51 mm, 52 mm, 53 mm, 65 mm, 66 mm or 67 mm.

The present invention is directed to a measuring tool for assessing the stomal and/or peristomal configuration of a patient comprising a plurality of the ostomy guide plates according to the present disclosure, wherein each plate is provided with at least one distinct dimensional parameter. I.e. a set of convex guide plates (i.e. the tool) should preferably contain varying sizes and preferably be easy to carry around. I.e. the ostomy guide plates in a convex measuring tool are different, e.g. like a set of measuring spoons. One exemplary convex measuring tool according to the disclosure comprises six ostomy guide plates with aperture diameters of 23, 30, 35, 40, 45 and 50 mm. Another exemplary convex measuring tool according to the disclosure comprises four ostomy guide plates with aperture diameters of 23, 30, 42 and 59 mm.

The depth of the convex protrusion in a range of convex products from the same manufacturer is typically fixed. Thus, in the preferred embodiment each ostomy guide plate is provided with a similar depth of the convex protrusion, e.g. a depth of 4 mm, 5 mm or 6 mm.

In the preferred embodiment each ostomy guide plate in a convex measuring tool is provided with a distinct aperture diameter. Thus, the present convex measuring tool can provide the nurse with a range of aperture diameters to better assess the diameter of the stoma and select the correct ostomy product. Each ostomy guide plate in a measuring tool may be provided with a predefined colour coding, e.g. a different colour mark on the guide for each aperture diameter. Each colour mark may then correspond to the colour marks of one or more ostomy products. Thus, when the nurse has determined which ostomy guide plate in the measuring tool that best suits the patient, the colour code on the guide plate tells the nurse which ostomy product, or which range of ostomy products, will best suit the patient.

In one embodiment each ostomy guide plate in a convex measuring tool is provided with a distinct diameter of the resting plateau. In another embodiment a convex measuring tool is provided with ostomy guide plates representing at least two, three, five or six, preferably four, different diameters of the resting plateau. Thus, the present convex measuring tool can provide the nurse with a range of sizes of the resting plateau thereby representing the range of plateau diameters which is typically available from convex products from the same manufacturer.

In one embodiment each ostomy guide plate in a convex measuring tool is provided with a distinct outer diameter of the plate. In another embodiment a convex measuring tool is provided with ostomy guide plates representing at least two, three, five or six, preferably four, different outer diameters of the plate.

In one embodiment the ostomy guide plates of a convex measuring tool are adapted to be attached together like a set of measuring spoons. In a further embodiment the ostomy guide plates of a convex measuring tool are adapted to be stackable. This may be provided to reduce the 3D footprint of the device thereby reducing storage space and making transport easier.

A range of convex ostomy pouching systems from a single manufacturer may comprise a range of diameters of the connection ring of the baseplate which provides the secure connection to the pouch. Each baseplate ring diameter is typically provided with a specific diameter of the convex resting plateau but may be provided with a range of pre-cut diameters of the aperture. Thus, for the present convex measuring tool to represent the convex product range from a manufacturer it may be advantageous if two or more ostomy guide plates can be combined in use. Thus, in one embodiment two or more ostomy guide plates from a convex measuring tool may be adapted to be interlocked, such as pairwise interlocked, preferably such that the apertures are centred. In another embodiment at least two ostomy guide plates from a convex measuring tool are provided with means for arranging the first ostomy guide plate on top of the second ostomy guide plate, preferably such that the apertures are centred.

A further aspect of the disclosure relates to a method for assessing the need of convexity when selecting an ostomy pouching system for a patient with a colostomy. In case the stoma does not sufficiently protrude beyond the abdominal skin surface of the patient or if the muscles surrounding the stoma lack rigidity, a convex solution might be the correct choice but the nurse cannot evaluate the stomal / peristomal configuration. The solution is to apply the present ostomy guide plate on the patient to test the case of applying pressure against the patient's abdomen by means of the ostomy guide plate to see if that cause the stoma to protrude, however without causing too much discomfort to the patient. Thus, a further aspect of the disclosure relates to a method for assessing the need of convexity when selecting an ostomy pouching system for a patient with a colostomy, said method comprising the steps of
a) disposing an ostomy guide plate according to the present disclosure on the abdominal skin of the patient with the aperture of the plate positioned over the stoma, and
b) applying pressure by hand to the distal face of the ostomy guide plate to assess the demand for abdominal pressure and stomal / peristomal support.

Once it has been determined whether a convex product is the correct choice the next step may be to select the correct size ostomy pouching system without bringing the entire product range to the bedside. Thus, a further embodiment relates to a method for selecting an ostomy pouching system for a patient with a colostomy, the method comprising the steps of:
a) disposing an ostomy guide plate selected from a measuring tool according to the present disclosure on the abdominal skin of the patient with the aperture of the plate positioned over the stoma,
b) evaluating the dimensions of the ostomy guide plate with regard to the stomal and peristomal anatomy of the patient,
c) optionally selecting a different ostomy guide plate from the measuring tool and repeating steps a) and b),
d) optionally applying pressure by hand to the distal face of the ostomy guide plate to assess the demand for a convex ostomy product, and
e) selecting the ostomy pouching system that accommodates the stomal and peristomal anatomy of the patient.

### Description of Drawings

The disclosure will now be described in further details with reference to the drawings showing exemplary embodiments.
- Fig. 1a: is a perspective illustration of one embodiment of the ostomy guide plate,
- Fig. 1b: is a perspective illustration of one embodiment of the measuring tool comprising a plurality of the guide plates from fig. 1a,
- Fig. 1c: is a top view schematic illustration of the collection of ostomy guide plates in the measuring tool in fig. 1b,
- Fig. 2a-c: shows further embodiments of the ostomy guide plate and the measuring tool,
- Fig. 3a-c: shows further embodiments of the ostomy guide plate and the measuring tool,
- Fig. 4a-c: shows further embodiments of the ostomy guide plate and the measuring tool, and
- Fig. 5a-e: shows examples of packing, storing and transportation of a convex measuring tool.

### Detailed description

Firm convexity is the most common for convexity products. Most firm convex products are constructed with a round rigid plastic molded insert inside the skin barrier to provide pressure and support to the peristomal area. These inserts vary from manufacturer to manufacturer in style, shape, depth and gradient.

The role of convexity is to
- Provide a mirror image of or to support the peristomal field
- Allow for continuous contact between the skin and the pouching system to create a secure seal
- Create pressure on the peristomal skin leading to stomal protrusion into the pouch whereby contents go into the pouch and not between the barrier and the skin

The right convex product may
- Reduce the number of accessories utilized for e.g. treatment of sore skin
- Simplify the stoma appliance process, e.g. one convex appliance rather than pastes, washers, etc.
- Help solve problematic stomal cases to avoid leakage and other discomforts

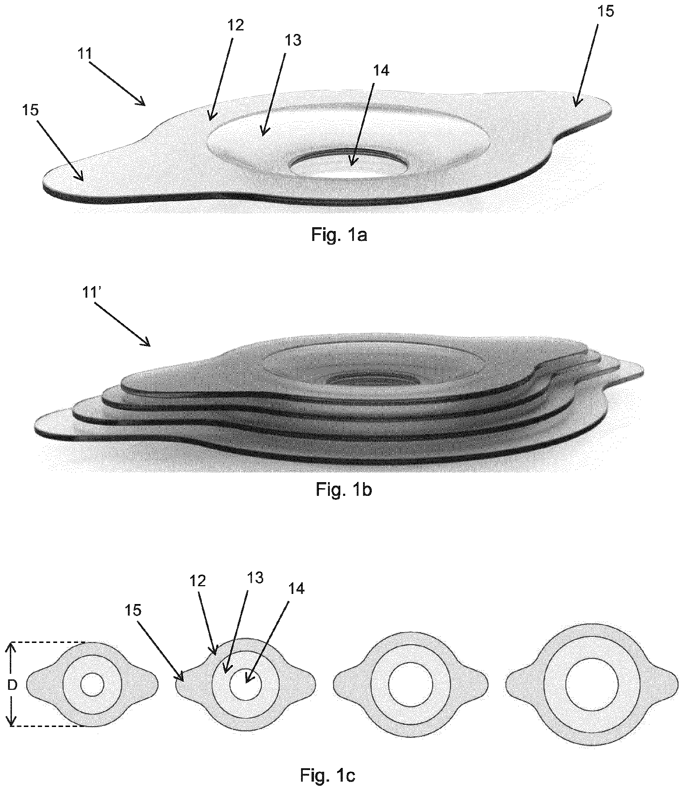

Fig. 1a is a perspective illustration of a preferred embodiment of the ostomy guide plate 11 according to the disclosure. Only the distal face of the plate is visible with the plane distal surface 12, the concave depression 13 corresponding to the convex protrusion of the proximal side of the plate 11. The circular concave depression 13 is located centrally in the plate 11 and the circular aperture 14 is located centrally in the concave depression 13. The illustrated guide plate 11 is manufactured in a rigid transparent plastic material and has an overall thickness of about 2 mm. The guide plate 11 is provided with two oppositely located handles 15 which are formed as natural extensions of the plane distal surface and with the same thickness. In use a nurse can apply pressure to the two handles 15, when assessing the need of convexity and when assessing the stomal configuration of a patient, thereby applying an equally distributed pressure to the guide plate 11. Besides the handles the outer circumference of the guide plate 11 is circular.

Fig. 1b is a perspective illustration of four of the ostomy guide plates 11 shown in fig. 1a stacked on top of each other thereby forming a measuring tool 11' according to one embodiment. As seen from the figure the guide plates in the tool 11' are of different size and they are stackable. The outer diameter, the diameter of the convex protrusion, the diameter of the resting plateau and the diameter of the aperture is increasing for the tool 11' measuring the guide plates from the bottom and up. This is better visualised in the top view schematic drawings in fig. 1c where the outer diameter D of the leftmost guide plate is illustrated.

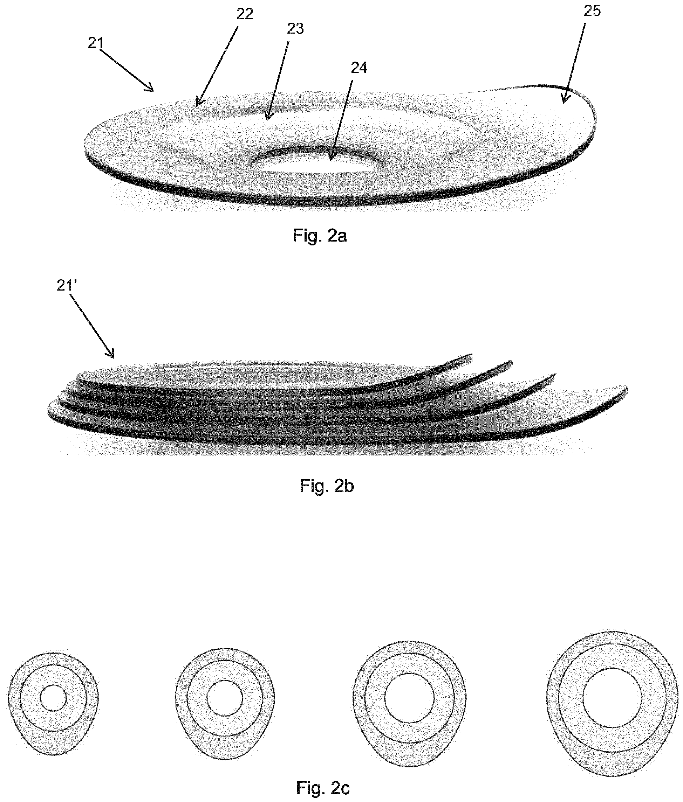

Figs. 2a-c corresponds to figs. 1a-c in nomenclature and show other embodiments of the guide plate 21 and the measuring tool 21'. One difference is that the guide plate 21 is provided with a single handle 25 which points upwards compared to the plane distal surface 22. This may make the guide plate easier to grab for a nurse and place on the skin of a patient with only one hand, possibly without touching the skin of the patient with the hand.

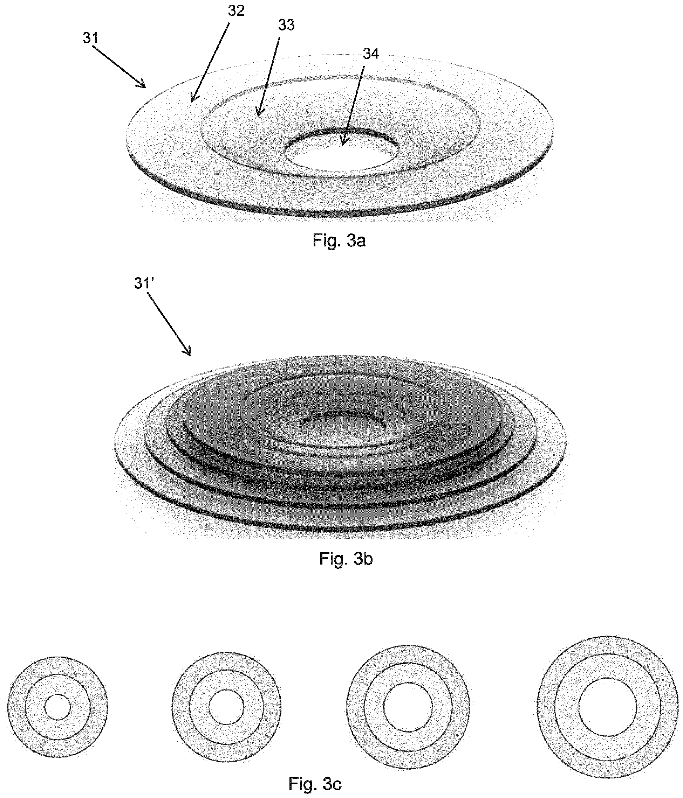

Figs. 3a-c corresponds to figs. 1a-c and 2a-c in nomenclature and show other embodiments of the guide plate 31 and the measuring tool 31'. One difference is that the guide plate 31 is not provided with a specific handle. The distal surface "disc" 32 can then function as a handle. This may reduce the footprint of the guide plate 31 and the measuring tool 31'.

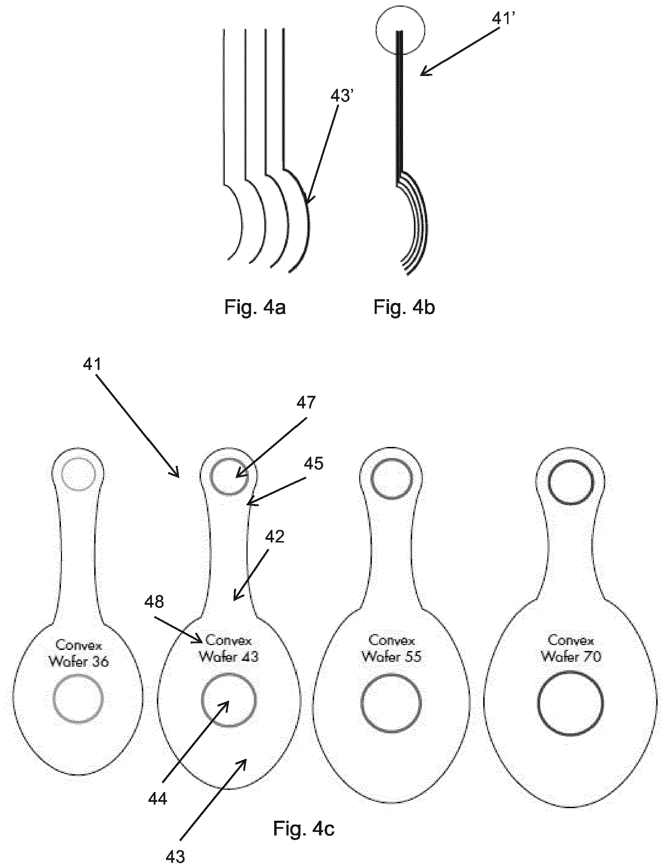

Fig. 4a-c shows yet another embodiment of an ostomy guide plate 41 and the corresponding measuring tool 41'. This embodiment resembles a classical measuring spoon with the plane distal surface 42 functioning as the handle 45 with a concave depression 43 corresponding to the convex protrusion 43' and an aperture 44. This embodiment of the guide plate 41 is provided with an additional aperture 47 in the handle (like a measuring spoon) for the guide plates to be attached together to form the measuring tool 41'. A visible marking 48 is provided on the distal face of the guide plate 41 with the number indicating the diameter of connecting ring of the baseplate (wafer) of the corresponding ostomy product.

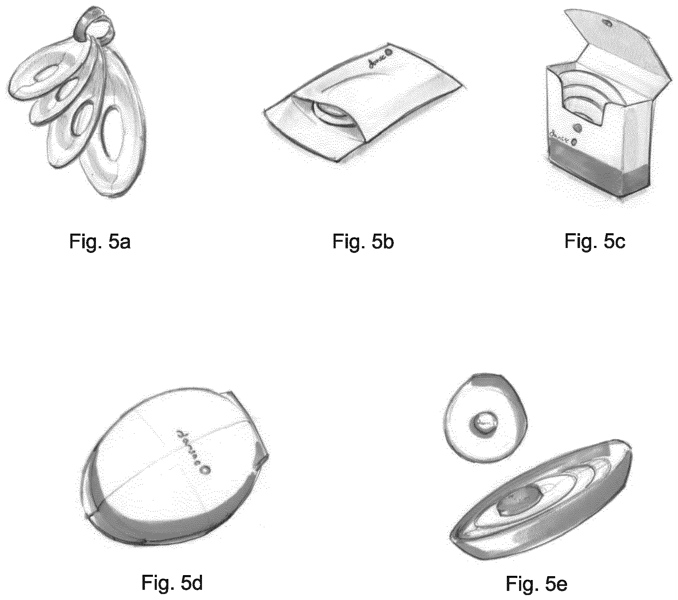

Fig. 5a shows an example of how ostomy guide plates can be attached together to form a convex measuring tool. The ostomy guide plates and the convex measuring tool may be one-time use disposable products. However, preferably they are standard reusable tools for a nurse and must thus be transported and stored safely and hygienic and they must be disinfectionable / sustainable to disinfectants due to the possible contact with vulnerable surgical abdominal areas of the patient. Examples for storing and transport of the convex measuring tool are illustrated in figs. 5b-e with an envelope like case as shown in fig. 5b or in box like cases as shown in fig. 5c, 5d and 5e.

## Claims

1. A measuring tool (11', 21', 31', 41') for assessing the stomal and/or peristomal configuration of a patient comprising a plurality of ostomy guide plates (11, 21, 31, 41), each ostomy guide plate comprising a distal face (12, 22, 32, 42), a proximal face with a convex protrusion and an aperture (14, 24, 34, 44) in said convex protrusion, wherein the surface profile of each convex protrusion is a smooth and/or rounded curve, wherein each ostomy guide plate (11, 21, 31, 41) is adapted for non-binding disposition on the skin of a patient with the aperture (14, 24, 34, 44) positioned over the stoma when assessing the stomal and/or peristomal configuration of the patient, and wherein for each ostomy guide plate (11, 21, 31, 41) the diameter of the aperture, the outer diameter, and/or the diameter of the convex protrusion is different.

2. The measuring tool (11', 21', 31', 41') according to claim 1, wherein each ostomy guide plate is translucent or wherein at least a part of each ostomy guide plate is transparent.

3. The measuring tool (11', 21', 31', 41') according to any of preceding claims, wherein the proximal face (12, 22, 32, 42) of each ostomy guide plate (11, 21, 31, 41) is not provided with an adhesive layer.

4. The measuring tool (11', 21', 31', 41') according to any of preceding claims, wherein the distal side (12, 22, 32, 42) of each ostomy guide plate is provided with a concave depression (13, 23, 33, 43) corresponding to the convex protrusion.

5. The measuring tool (11', 21', 31', 41') according to any of preceding claims, wherein the diameter of the apertures, the outer diameters, the depth of the convex protrusions, and/or the diameter of the convex protrusions are predefined.

6. The measuring tool (11', 21', 31', 41') according to claim 5, further comprising a colour coding corresponding to the predefined value of the apertures, the outer diameters, the depth of the convex protrusions, and/or the diameter of the convex protrusions.

7. The measuring tool (11', 21', 31', 41') according to any of preceding claims, wherein each convex protrusion is provided with a substantially plane resting plateau, preferably with a predefined diameter of each resting plateau.

8. The measuring tool (11', 21', 31', 41') according to any of preceding claims, wherein each ostomy guide plate comprises at least one visible mark (48) for indicating at least one dimensional parameter.

9. The measuring tool (11', 21', 31', 41') according to any of preceding claims, wherein each ostomy guide plate comprises one or more handles (15, 25, 35, 45).

10. The measuring tool (11', 21', 31', 41') according to any of preceding claims, wherein the depth of each convex protrusion is between 1 and 10 mm, such as approx. 1, 2, 3, 4, 5, 6, 7, 8, 9 or 10 mm, preferably 6 mm.

11. The measuring tool (11', 21', 31', 41') according to any of preceding claims, wherein each ostomy guide plate is provided with equal depths of the convex protrusion.

12. The measuring tool (11', 21', 31', 41') according to any of preceding claims, wherein each ostomy guide plate is provided with a predefined colour coding, and/or wherein each ostomy guide plate is provided with a predefined colour coding corresponding to the aperture diameter.

13. The measuring tool (11', 21', 31', 41') according to any of preceding claims, wherein the ostomy guide plates (11, 21, 31, 41) are adapted to be stackable, and/or wherein the ostomy guide plates are adapted to be interlocked, such as pairwise interlocked.

14. The measuring tool according to any of preceding claims, comprising six ostomy guide plates with aperture diameters of 23, 30, 35, 40, 45 and 50 mm.

15. The measuring tool according to any of preceding claims, comprising four ostomy guide plates with aperture diameters of 23, 30, 42, and 59 mm.

## Patentansprüche

1. Messwerkzeug (11', 21', 31', 41') zum Beurteilen der stomalen und/oder peristomalen Konfiguration eines Patienten, das eine Vielzahl von Ostomieführungsplatten (11, 21, 31, 41) umfasst, wobei jede Ostomieführungsplatte eine distale Fläche (12, 22, 32, 42), eine proximale Fläche mit einem konvexen Vorsprung und eine Öffnung (14, 24, 34, 44) in dem konvexen Vorsprung umfasst, wobei das Oberflächenprofil jedes konvexen Vorsprungs eine glatte und/oder abgerundete Kurve ist, wobei jede Ostomieführungsplatte (11, 21, 31, 41) für eine nicht bindende Anordnung auf der Haut eines Patienten ausgelegt ist, wobei die Öffnung (14, 24, 34, 44) über dem Stoma angeordnet ist, wenn die stomale und/oder peristomale Konfiguration des Patienten beurteilt wird, und wobei für jede Ostomieführungsplatte (11, 21, 31, 41) der Durchmesser der Öffnung, der Außendurchmesser und/oder der Durchmesser des konvexen Vorsprungs unterschiedlich ist.

2. Messwerkzeug (11', 21', 31', 41') nach Anspruch 1, wobei jede Ostomieführungsplatte lichtdurchlässig ist oder wobei mindestens ein Teil jeder Ostomieführungsplatte transparent ist.

3. Messwerkzeug (11', 21', 31', 41') nach einem der vorhergehenden Ansprüche, wobei die proximale Fläche (12, 22, 32, 42) jeder Ostomieführungsplatte (11, 21, 31, 41) nicht mit einer Klebschicht versehen ist.

4. Messwerkzeug (11', 21', 31', 41') nach einem der vorhergehenden Ansprüche, wobei die distale Seite (12, 22, 32, 42) jeder Ostomieführungsplatte mit einer konkaven Vertiefung (13, 23, 33, 43) entsprechend dem konvexen Vorsprung versehen ist.

5. Messwerkzeug (11', 21', 31', 41') nach einem der vorhergehenden Ansprüche, wobei der Durchmesser der Öffnungen, die Außendurchmesser, die Tiefe der konvexen Vorsprünge und/oder der Durchmesser der konvexen Vorsprünge vordefiniert sind.

6. Messwerkzeug (11', 21', 31', 41') nach Anspruch 5, ferner umfassend eine Farbkodierung, die dem vorgegebenen Wert der Öffnungen, der Außendurchmesser, der Tiefe der konvexen Vorsprünge und/oder des Durchmessers der konvexen Vorsprünge entspricht.

7. Messwerkzeug (11', 21', 31', 41') nach einem der vorhergehenden Ansprüche, wobei jeder konvexe Vorsprung mit einem im Wesentlichen ebenen Ruheplateau, vorzugsweise mit einem vorgegebenen Durchmesser jedes Ruheplateaus, versehen ist.

8. Messwerkzeug (11', 21', 31', 41') nach einem der vorhergehenden Ansprüche, wobei jede Ostomieführungsplatte mindestens eine sichtbare Markierung (48) zum Anzeigen mindestens eines Dimensionsparameters aufweist.

9. Messwerkzeug (11', 21', 31', 41') nach einem der vorhergehenden Ansprüche, wobei jede Ostomieführungsplatte einen oder mehrere Griffe (15, 25, 35, 45) umfasst.

10. Messwerkzeug (11', 21', 31', 41') nach einem der vorhergehenden Ansprüche, wobei die Tiefe jedes konvexen Vorsprungs zwischen 1 und 10 mm beträgt, wie beispielsweise ca. 1, 2, 3, 4, 5, 6, 7, 8, 9 oder 10 mm, vorzugsweise 6 mm.

11. Messwerkzeug (11', 21', 31', 41') nach einem der vorhergehenden Ansprüche, wobei jede Ostomieführungsplatte mit gleichen Tiefen des konvexen Vorsprungs versehen ist.

12. Messwerkzeug (11', 21', 31', 41') nach einem der vorhergehenden Ansprüche, wobei jede Ostomieführungsplatte mit einer vorgegebenen Farbkodierung versehen ist, und/oder wobei jede Ostomieführungsplatte mit einer vorgegebenen Farbkodierung entsprechend dem Öffnungsdurchmesser versehen ist.

13. Messwerkzeug (11', 21', 31', 41') nach einem der vorhergehenden Ansprüche, wobei die Ostomieführungsplatten (11, 21, 31, 41) ausgebildet sind, um stapelbar zu sein, und/oder wobei die Ostomieführungsplatten verriegelt, wie beispielsweise paarweise verriegelt, ausgebildet sind.

14. Messwerkzeug nach einem der vorhergehenden Ansprüche, umfassend sechs Ostomieführungsplatten mit Öffnungsdurchmessern von 23, 30, 35, 40, 45 und 50 mm.

15. Messwerkzeug nach einem der vorhergehenden Ansprüche, umfassend vier Ostomieführungsplatten mit Öffnungsdurchmessern von 23, 30, 42 und 59 mm.

## Revendications

1. Outil de mesure (11', 21', 31', 41') pour évaluer la configuration stomiale et/ou péristomiale d'un patient comprenant une pluralité de plaques de guidage de stomie (11, 21, 31, 41), chaque plaque de guidage de stomie comprenant une face distale (12, 22, 32, 42), une face proximale avec une protubérance convexe et une ouverture (14, 24, 34, 44) dans ladite protubérance convexe, dans lequel le profil de surface de chaque protubérance convexe est une courbe lisse et/ou arrondie, dans lequel chaque plaque de guidage de stomie (11, 21, 31, 41) est adaptée pour une disposition de non-liaison sur la peau d'un patient avec l'ouverture (14, 24, 34, 44) positionnée au-dessus de la stomie lors de l'évaluation de la configuration stomiale et/ou péristomiale du patient, et dans lequel, pour chaque plaque de guidage de stomie (11, 21, 31, 41), le diamètre de l'ouverture, le diamètre extérieur et/ou le diamètre de la protubérance convexe est différent.

2. Outil de mesure (11', 21', 31', 41') selon la revendication 1, dans lequel chaque plaque de guidage de stomie est translucide ou dans lequel au moins une partie de chaque plaque de guidage de stomie est transparente.

3. Outil de mesure (11', 21', 31', 41') selon l'une quelconque des revendications précédentes, dans lequel la face proximale (12, 22, 32, 42) de chaque plaque de guidage de stomie (11, 21, 31, 41) n'est pas pourvue d'une couche adhésive.

4. Outil de mesure (11', 21', 31', 41') selon l'une quelconque des revendications précédentes, dans lequel le côté distal (12, 22, 32, 42) de chaque plaque de guidage de stomie est pourvu d'une dépression concave (13, 23, 33, 43) correspondant à la protubérance convexe.

5. Outil de mesure (11', 21', 31', 41') selon l'une quelconque des revendications précédentes, dans lequel le diamètre des ouvertures, les diamètres extérieurs, la profondeur des protubérances convexes et/ou le diamètre des protubérances convexes sont prédéfinis.

6. Outil de mesure (11', 21', 31', 41') selon la revendication 5, comprenant en outre un code couleur correspondant à la valeur prédéfinie des ouvertures, des diamètres extérieurs, de la profondeur des protubérances convexes et/ou du diamètre des protubérances convexes.

7. Outil de mesure (11', 21', 31', 41') selon l'une quelconque des revendications précédentes, dans lequel chaque protubérance convexe est pourvue d'un plateau de repos sensiblement plat, de préférence avec un diamètre prédéfini de chaque plateau de repos.

8. Outil de mesure (11', 21', 31', 41') selon l'une quelconque des revendications précédentes, dans lequel chaque plaque de guidage de stomie comprend au moins une marque visible (48) pour indiquer au moins un paramètre dimensionnel.

9. Outil de mesure (11', 21', 31', 41') selon l'une quelconque des revendications précédentes, dans lequel chaque plaque de guidage de stomie comprend une ou plusieurs poignées (15, 25, 35, 45).

10. Outil de mesure (11', 21', 31', 41') selon l'une quelconque des revendications précédentes, dans lequel la profondeur de chaque protubérance convexe est comprise entre 1 et 10 mm, par exemple, approximativement 1, 2, 3, 4, 5, 6, 7, 8, 9 ou 10 mm, de préférence 6 mm.

11. Outil de mesure (11', 21', 31', 41') selon l'une quelconque des revendications précédentes, dans lequel chaque plaque de guidage de stomie est pourvue de profondeurs égales de la protubérance convexe.

12. Outil de mesure (11', 21', 31', 41') selon l'une quelconque des revendications précédentes, dans lequel chaque plaque de guidage de stomie est pourvue d'un code couleur prédéfini et/ou dans lequel chaque plaque de guidage de stomie est pourvue d'un code couleur prédéfini correspondant au diamètre d'ouverture.

13. Outil de mesure (11', 21', 31', 41') selon l'une quelconque des revendications précédentes, dans lequel les plaques de guidage de stomie (11, 21, 31, 41) sont adaptées pour être empilables et/ou dans lequel les plaques de guidage de stomie sont adaptées pour être imbriquées, par exemple imbriquées par paires.

14. Outil de mesure selon l'une quelconque des revendications précédentes, comprenant six plaques de guidage de stomie avec des diamètres d'ouverture de 23, 30, 35, 40, 45 et 50 mm.

15. Outil de mesure selon l'une quelconque des revendications précédentes, comprenant quatre plaques de guidage de stomie avec des diamètres d'ouverture de 23, 30, 42 et 59 mm.
